# EUROPEAN PATENT SPECIFICATION

(11) **EP 2 906 294 B1**
(45) Date of publication and mention of the grant of the patent: **18.04.2018**
(21) Application number: 13762992.9
(22) Date of filing: 13.09.2013
(51) Int. Cl.: A61P 35/00, A61K 31/44, A61K 31/506

(54) **COMBINATION OF A 6-OXO-1,6-DIHYDRO-PYRIDAZINE DERIVATIVE HAVING ANTI-CANCER ACTIVITY WITH A MEK INHIBITOR**
KOMBINATION AUS EINEM 6-OXO-1,6-DIHYDROPYRIDAZINDERIVAT MIT WIRKUNG GEGEN KREBS UND EINEM MEK-HEMMER
ASSOCIATION D'UN DÉRIVÉ DE 6-OXO-1,6-DIHYDRO-PYRIDAZINE PRÉSENTANT UNE ACTIVITÉ ANTICANCÉREUSE À UN INHIBITEUR DE MEK

(30) Priority: 11.10.2012 EP 12007039
(43) Date of publication of application: 19.08.2015
(73) Proprietor: Merck Patent GmbH, 64293 Darmstadt (DE)
(72) Inventor: BLADT, Friedhelm, 69120 Heidelberg (DE); FRIESE-HAMIM, Manja, 64546 Moerfelden-Walldorf (DE)
(86) International application number: PCT/EP2013/002747
(87) International publication number: WO 2014/056566

(56) References cited:
- WO-A1-2009/007074
- WO-A1-2012/078832
- FRIEDHELM BLADT1, ANDREE BLAUKAT1, BLADT F., BLAUKAT A., DORSCH D., FRIESE-HAMIM M., MEYRING M., SCHADT O., STIEBER F.: "Abstract 1787: The c-Met inhibitors EMD 1214063 and EMD 1204831 are effective in combination with EGFR and VEGF inhibitors in NSCLC models", CANCER RESEARCH, [Online] vol. 72, no. 8 Supp 1, 15 April 2012 (2012-04-15), XP002715245, ISSN: 0008-5472, DOI: 10.1158/1538-7445.AM2012-1787 internet Retrieved from the Internet: URL:http://cancerres.aacrjournals.org/cgi/ content/short/72/8_MeetingAbstracts/1787?r ss=1> [retrieved on 2013-10-23]
- LEONG STEPHEN ET AL: "Novel agents in the treatment of metastatic colorectal cancer.", CANCER JOURNAL (SUDBURY, MASS.) 2010 MAY-JUN, vol. 16, no. 3, May 2010 (2010-05), pages 273-282, XP002715246, ISSN: 1540-336X
- DATABASE WPI Week 201024 Thomson Scientific, London, GB; AN 2010-C60981 XP002715247, -& CN 101 653 607 A (DINGHONG INT INVESTMENT HONGKONG CO LTD) 24 February 2010 (2010-02-24)
- DAMARAJU VIJAYA L ET AL: "Synergistic activity of troxacitabine (Troxatyl(TM)) and gemcitabine in pancreatic cancer", BMC CANCER, BIOMED CENTRAL, LONDON, GB, vol. 7, no. 1, 3 July 2007 (2007-07-03), page 121, XP021029093, ISSN: 1471-2407, DOI: 10.1186/1471-2407-7-121

## Description

### FIELD OF THE INVENTION

This invention relates to a pharmaceutical composition for cancer disease, which comprises a compound having anti-cancer activity, namely 3-(1-{3-[5-(1-Methyl-piperidin-4-ylmethoxy)-pyrimidin-2-yl]-benzyl)-6-oxo-1,6-dihydro-pyridazin-3-yl)-benzonitrile or a pharmaceutically acceptable salt and/or solvate thereof in combination with a MEK inhibitor, namely N-((S)-2,3-dihydroxypropyl)-3-(2-fluoro-4-iodo-phenylamino)-isonicotinamide or a pharmaceutically acceptable salt and/or solvate thereof.

### BACKGROUND OF THE INVENTION

The invention had the object of finding novel pharmaceutical compositions having valuable properties, in particular those which can be used for the preparation of medicaments.

Moreover, aim of this invention are new compositions for the prevention and treatment of neoplastic malignancies including, solid tumor cancers, cancers of the lymphatic or blood system.

It has been found that the pharmaceutical compositions according to the invention and pharmaceutically acceptable salts and/or solvates thereof have very valuable pharmacological properties while being well tolerated.

Targeted therapies selectively inhibit specific targets in tumors. By combining these targeted therapies with standard of cares (SoC) the activity of the SoC can be improved. It has been found that by combining 3-(1-{3-[5-(1-Methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof with N-((S)-2,3-dihydroxy-propyl)-3-(2-fluoro-4-iodo-phenylamino)-isonicotinamide or a pharmaceutically acceptable salt and/or solvate thereof, the activity of anti-cancer compounds in xenografts is improved.

The efficacy in NSCLC (non-small cell lung cancer) xenograft models is enhanced compared to monotherapies. The enhanced efficacy in the combination group is observed without increase in toxicity.

### PRIOR ART

3-(1-{3-[5-(1-Methyl-piperidin-4-ylmethoxy)-pyrimidin-2-yl]-benzyl}-6-oxo-1,6-dihydro-pyridazin-3-yl)-benzonitrile has been described in WO 2009/006959 A1.

3-(1-{3-[5-(1-Methyl-piperidin-4-ylmethoxy)-pyrimidin-2-yl]-benzyl}-6-oxo-1,6-dihydro-pyridazin-3-yl)-benzonitrile hydrochloride hydrate has been described in WO 2009/007074 A1.

A combination of the MEK inhibitor N-((S)-2,3-dihydroxy-propyl)-3-(2-fluoro-4-iodo-phenylamino)-isonicotinamide with a PI3K inhibitor is described in WO 2012/078832 A1.

### SUMMARY OF THE INVENTION

The invention relates to a composition comprising 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof and N-((S)-2,3-dihydroxy-propyl)-3-(2-fluoro-4-iodo-phenylamino)-isonicotinamide or a pharmaceutically acceptable salt and/or solvate thereof.

Moreover, the invention relates to a composition comprising 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate and N-((S)-2,3-dihydroxypropyl)-3-(2-fluoro-4-iodo-phenylamino)-isonicotinamide or a pharmaceutically acceptable salt and/or solvate thereof.

Moreover, the invention relates to a composition of 3-(1-{3-[5-(1-methyl-piperidin-4-ylmethoxy)-pyrimidin-2-yl]-benzyl)-6-oxo-1,6-dihydro-pyridazin-3-yl)-benzonitrile or a pharmaceutically acceptable salt and/or solvate thereof and N-((S)-2,3-dihydroxy-propyl)-3-(2-fluoro-4-iodo-phenylamino)-isonicotinamide or a pharmaceutically acceptable salt and/or solvate thereof, for the use for the treatment of diseases selected from the group
cancer of the head, neck, eye, mouth, throat, esophagus, bronchus, larynx, pharynx, chest, bone, lung, colon, rectum, stomach, prostate, urinary bladder, uterine, cervix, breast, ovaries, testicles or other reproductive organs, skin, thyroid, blood, lymph nodes, kidney, liver, pancreas, brain, central nervous system, solid tumors and blood-borne tumors.

Moreover, the invention relates to 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof for the use as a medicament for the treatment of cancer, selected from the group colorectal, lung, breast, kidney, and glioblastomas,
wherein the medicament is to be used in combination with N-((S)-2,3-dihydroxy-propyl)-3-(2-fluoro-4-iodo-phenylamino)-isonicotinamide or a pharmaceutically acceptable salt and/or solvate thereof.

Moreover, the invention relates to 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate for the use as a medicament for the treatment of cancer, selected from the group colorectal, lung, breast, kidney, and glioblastomas,
wherein the medicament is to be used in combination with N-((S)-2,3-dihydroxy-propyl)-3-(2-fluoro-4-iodo-phenylamino)-isonicotinamide or a pharmaceutically acceptable salt and/or solvate thereof.

Moreover, the invention relates to 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof for the use as a medicament for the treatment of cancer, selected from the group small cell lung cancer (SCLC), non-small cell lung cancer (NSCLC), squamous cell cancer of the head and neck (SCCHN),
wherein the medicament is to be used in combination with N-((S)-2,3-dihydroxy-propyl)-3-(2-fluoro-4-iodo-phenylamino)-isonicotinamide or a pharmaceutically acceptable salt and/or solvate thereof.
Moreover, the invention relates to the use as described above,
wherein 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof or
3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate
is administered to a patient in an amount of 250 mg to 12500 mg per week, preferably in an amount of 800 mg to 8000 mg per week, particularly preferably in an amount of 500 mg to 2000 mg per week.

According to the present invention therapeutically active compositions may also be provided by means of a pharmaceutical kit comprising a package comprising 3-(1-{3-[5-(1-methyl-piperidin-4-ylmethoxy)-pyrimidin-2-yl]-benzyl]-6-oxo-1,6-dihydro-pyridazin-3-yl)-benzonitrile or a pharmaceutically acceptable salt and/or solvate thereof, and N-((S)-2,3-dihydroxy-propyl)-3-(2-fluoro-4-iodo-phenylamino)-isonicotinamide or a pharmaceutically acceptable salt and/or solvate thereof, in single packages or in separate containers.

The therapy with these combinations may include optionally further treatment with radiation. The invention relates furthermore to a new therapy form comprising the start of the administration of 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof prior to radiotherapy.
In this new therapy form comprising the start of the administration of 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof prior to radiotherapy, it is a preferred feature that the 3-(1-{3-[5-(1-methyl-piperidin-4-ylmethoxy)-pyrimidin-2-ylj-benzyl}-6-oxo-1,6-dihydro-pyridazin-3-yl)-benzonitrile or a pharmaceutically acceptable salt and/or solvate thereof is administered prior and/or during the administration of the further cancer cotherapeutic agent, preferably at least during a significant part of the treatment regimen. In this context, according to the present invention, radiation, or, radiotherapy preferably has to be understood as a cancer cotherapeutic agent.

The invention also relates to the optically active forms (stereoisomers), the enantiomers, the racemates, the diastereomers and the hydrates and solvates of these compounds.
The invention also relates to the solvates of the salts of the compounds e.g. the mono- or dihydrate of the hydrochloride.

The term solvates of the compounds is taken to mean adductions of inert solvent molecules onto the compounds which form owing to their mutual attractive force. Solvates are, for example, mono- or dihydrates or alcoholates.

The expression "effective amount" denotes the amount of a medicament or of a pharmaceutical active ingredient which causes in a tissue, system, animal or human a biological or medical response which is sought or desired, for example, by a researcher or physician.
In addition, the expression "therapeutically effective amount" denotes an amount which, compared with a corresponding subject who has not received this amount, has the following consequence:
improved treatment, healing, prevention or elimination of a disease, syndrome, condition, complaint, disorder or side-effects or also the reduction in the advance of a disease, complaint or disorder.
The expression "therapeutically effective amount" also encompasses the amounts which are effective for increasing normal physiological function.

### Pharmaceutical salts and other forms

The said compounds according to the invention can be used in their final non-salt form. On the other hand, the present invention also encompasses the use of these compounds in the form of their pharmaceutically acceptable salts, which can be derived from various organic and inorganic acids and bases by procedures known in the art. Pharmaceutically acceptable salt forms of 3-(1-{3-[5-(1-methyl-piperidin-4-ylmethoxy)-pyrimidin-2-yl]-benzyl}-6-oxo-1,6-dihydro-pyridazin-3-yl)-benzonitrile and N-((S)-2,3-dihydroxy-propyl)-3-(2-fluoro-4-iodo-phenylamino)-isonicotinamide are for the most part prepared by conventional methods.

If a compound contains a carboxyl group, one of its suitable salts can be formed by reacting the compound with a suitable base to give the corresponding base-addition salt. Such bases are, for example, alkali metal hydroxides, including potassium hydroxide, sodium hydroxide and lithium hydroxide; alkaline earth metal hydroxides, such as barium hydroxide and calcium hydroxide; alkali metal alkoxides, for example potassium ethoxide and sodium propoxide; and various organic bases, such as piperidine, diethanolamine and N-methylglutamine. The aluminium salts of the compounds are likewise included. In the case of certain compounds acid-addition salts can be formed by treating these compounds with pharmaceutically acceptable organic and inorganic acids, for example hydrogen halides, such as hydrogen chloride, hydrogen bromide or hydrogen iodide, other mineral acids and corresponding salts thereof, such as sulfate, nitrate or phosphate, and alkyl- and monoarylsulfonates, such as ethanesulfonate, toluenesulfonate and benzenesulfonate, and other organic acids and corresponding salts thereof, such as acetate, trifluoroacetate, tartrate, maleate, succinate, citrate, benzoate, salicylate, ascorbate. Accordingly, pharmaceutically acceptable acid-addition salts of the compounds include the following: acetate, adipate, alginate, arginate, aspartate, benzoate, benzenesulfonate (besylate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate (from mucic acid), galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, isobutyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, palmoate, pectinate, persulfate, phenylacetate, 3-phenylpropionate, phosphate, phosphonate, phthalate. Furthermore, the base salts of the compounds according to the invention include aluminium, ammonium, calcium, copper, iron(III), iron(II), lithium, magnesium, manganese(III), manganese(II), potassium, sodium and zinc salts. Of the above-mentioned salts, preference is given to ammonium; the alkali metal salts sodium and potassium, and the alkaline earth metal salts calcium and magnesium. Salts of the compounds which are derived from pharmaceutically acceptable organic non-toxic bases include salts of primary, secondary and tertiary amines, substituted amines, also including naturally occurring substituted amines, cyclic amines, and basic ion exchanger resins, for example arginine, betaine, caffeine, chloroprocaine, choline, N,N'-dibenzylethylenediamine (benzathine), dicyclohexylamine, diethanolamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine and tris(hydroxymethyl)methylamine (tromethamine). Compounds of the present invention which contain basic nitrogen-containing groups can be quaternised using agents such as (C₁-C₄)alkyl halides, for example methyl, ethyl, isopropyl and tert-butyl chloride, bromide and iodide; di(C₁-C₄)alkyl sulfates, for example dimethyl, diethyl and diamyl sulfate; (C₁₀-C₁₈)alkyl halides, for example decyl, dodecyl, lauryl, myristyl and stearyl chloride, bromide and iodide; and aryl(C₁-C₄)alkyl halides, for example benzyl chloride and phenethyl bromide. Both water- and oil-soluble compounds according to the invention can be prepared using such salts.

The above-mentioned pharmaceutical salts which are preferred include acetate, trifluoroacetate, besylate, citrate, fumarate, gluconate, hemisuccinate, hippurate, hydrochloride, hydrobromide, isethionate, mandelate, meglumine, nitrate, oleate, phosphonate, pivalate, sodium phosphate, stearate, sulfate, sulfosalicylate, tartrate, thiomalate, tosylate and tromethamine. Particular preference is given to hydrochloride, dihydrochloride, hydrobromide, maleate, mesylate, phosphate, sulfate and succinate.

The acid-addition salts of basic compounds are prepared by bringing the free base form into contact with a sufficient amount of the desired acid, causing the formation of the salt in a conventional manner. The free base can be regenerated by bringing the salt form into contact with a base and isolating the free base in a conventional manner. The free base forms differ in a certain respect from the corresponding salt forms thereof with respect to certain physical properties, such as solubility in polar solvents; for the purposes of the invention, however, the salts otherwise correspond to the respective free base forms thereof.

As mentioned, the pharmaceutically acceptable base-addition salts of the compounds are formed with metals or amines, such as alkali metals and alkaline earth metals or organic amines. Preferred metals are sodium, potassium, magnesium and calcium. Preferred organic amines are N,N'-dibenzylethylenediamine, chloroprocaine, choline, diethanolamine, ethylenediamine, N-methyl-D-glucamine and procaine.

The base-addition salts of acidic compounds according to the invention are prepared by bringing the free acid form into contact with a sufficient amount of the desired base, causing the formation of the salt in a conventional manner. The free acid can be regenerated by bringing the salt form into contact with an acid and isolating the free acid in a conventional manner. The free acid forms differ in a certain respect from the corresponding salt forms thereof with respect to certain physical properties, such as solubility in polar solvents; for the purposes of the invention, however, the salts otherwise correspond to the respective free acid forms thereof.

If a compound according to the invention contains more than one group which is capable of forming pharmaceutically acceptable salts of this type, the invention also encompasses multiple salts. Typical multiple salt forms include, for example, bitartrate, diacetate, difumarate, dimeglumine, diphosphate, disodium and trihydrochloride. With regard to that stated above, it can be seen that the expression "pharmaceutically acceptable salt" in the present connection is taken to mean an active ingredient which comprises a compound in the form of one of its salts, in particular if this salt form imparts improved pharmacokinetic properties on the active ingredient compared with the free form of the active ingredient or any other salt form of the active ingredient used earlier. The pharmaceutically acceptable salt form of the active ingredient can also provide this active ingredient for the first time with a desired pharmacokinetic property which it did not have earlier and can even have a positive influence on the pharmacodynamics of this active ingredient with respect to its therapeutic efficacy in the body.

The invention furthermore relates to medicaments comprising at least one compound and/or pharmaceutically acceptable salts, solvates, tautomers and stereoisomers thereof, including mixtures thereof in all ratios, and optionally excipients and/or adjuvants.

Pharmaceutical formulations can be administered in the form of dosage units which comprise a predetermined amount of active ingredient per dosage unit. Such a unit can comprise, for example, 0.5 mg to 1 g, preferably 1 mg to 700 mg, particularly preferably 5 mg to 100 mg, of a compound according to the invention, depending on the condition treated, the method of administration and the age, weight and condition of the patient, or pharmaceutical formulations can be administered in the form of dosage units which comprise a predetermined amount of active ingredient per dosage unit. Preferred dosage unit formulations are those which comprise a daily dose or part-dose, as indicated above, or a corresponding fraction thereof of an active ingredient. Furthermore, pharmaceutical formulations of this type can be prepared using a process which is generally known in the pharmaceutical art.

Pharmaceutical formulations can be adapted for administration via any desired suitable method, for example by oral (including buccal or sublingual), rectal, nasal, topical (including buccal, sublingual or transdermal), vaginal or parenteral (including subcutaneous, intramuscular, intravenous or intradermal) methods. Such formulations can be prepared using all processes known in the pharmaceutical art by, for example, combining the active ingredient with the excipient(s) or adjuvant(s).

Pharmaceutical formulations adapted for oral administration can be administered as separate units, such as, for example, capsules or tablets; powders or granules; solutions or suspensions in aqueous or non-aqueous liquids; edible foams or foam foods; or oil-in-water liquid emulsions or water-in-oil liquid emulsions.

Thus, for example, in the case of oral administration in the form of a tablet or capsule, the active-ingredient component can be combined with an oral, non-toxic and pharmaceutically acceptable inert excipient, such as, for example, ethanol, glycerol, water. Powders are prepared by comminuting the compound to a suitable fine size and mixing it with a pharmaceutical excipient comminuted in a similar manner, such as, for example, an edible carbohydrate, such as, for example, starch or mannitol. A flavour, preservative, dispersant and dye may likewise be present.

Capsules are produced by preparing a powder mixture as described above and filling shaped gelatine shells therewith. Glidants and lubricants, such as, for example, highly disperse silicic acid, talc, magnesium stearate, calcium stearate or polyethylene glycol in solid form, can be added to the powder mixture before the filling operation. A disintegrant or solubiliser, such as, for example, agar-agar, calcium carbonate or sodium carbonate, may likewise be added in order to improve the availability of the medicament after the capsule has been taken.

In addition, if desired or necessary, suitable binders, lubricants and disintegrants as well as dyes can likewise be incorporated into the mixture. Suitable binders include starch, gelatine, natural sugars, such as, for example, glucose or beta-lactose, sweeteners made from maize, natural and synthetic rubber, such as, for example, acacia, tragacanth or sodium alginate, carboxymethylcellulose, polyethylene glycol, waxes. The lubricants used in these dosage forms include sodium oleate, sodium stearate, magnesium stearate, sodium benzoate, sodium acetate, sodium chloride and the like. The disintegrants include, without being restricted thereto, starch, methylcellulose, agar, bentonite, xanthan gum. The tablets are formulated by, for example, preparing a powder mixture, granulating or dry-pressing the mixture, adding a lubricant and a disintegrant and pressing the entire mixture to give tablets. A powder mixture is prepared by mixing the compound comminuted in a suitable manner with a diluent or a base, as described above, and optionally with a binder, such as, for example, carboxymethylcellulose, an alginate, gelatine or polyvinylpyrrolidone, a dissolution retardant, such as, for example, paraffin, an absorption accelerator, such as, for example, a quaternary salt, and/or an absorbant, such as, for example, bentonite, kaolin or dicalcium phosphate. The powder mixture can be granulated by wetting it with a binder, such as, for example, syrup, starch paste, acadia mucilage or solutions of cellulose or polymer materials and pressing it through a sieve. As an alternative to granulation, the powder mixture can be run through a tabletting machine, giving lumps of non-uniform shape, which are broken up to form granules. The granules can be lubricated by addition of stearic acid, a stearate salt, talc or mineral oil in order to prevent sticking to the tablet casting moulds. The lubricated mixture is then pressed to give tablets. The compounds according to the invention can also be combined with a free-flowing inert excipient and then pressed directly to give tablets without carrying out the granulation or dry-pressing steps. A transparent or opaque protective layer consisting of a shellac sealing layer, a layer of sugar or polymer material and a gloss layer of wax may be present. Dyes can be added to these coatings in order to be able to differentiate between different dosage units.

Oral liquids, such as, for example, solution, syrups and elixirs, can be prepared in the form of dosage units so that a given quantity comprises a pre-specified amount of the compound. Syrups can be prepared by dissolving the compound in an aqueous solution with a suitable flavour, while elixirs are prepared using a non-toxic alcoholic vehicle. Suspensions can be formulated by dispersion of the compound in a non-toxic vehicle. Solubilisers and emulsifiers, such as, for example, ethoxylated isostearyl alcohols and polyoxyethylene sorbitol ethers, preservatives, flavour additives, such as, for example, peppermint oil or natural sweeteners or saccharin, or other artificial sweeteners, can likewise be added.

The dosage unit formulations for oral administration can, if desired, be encapsulated in microcapsules. The formulation can also be prepared in such a way that the release is extended or retarded, such as, for example, by coating or embedding of particulate material in polymers, wax. The compounds and salts, solvates, tautomers and stereoisomers thereof can also be administered in the form of liposome delivery systems, such as, for example, small unilamellar vesicles, large unilamellar vesicles and multilamellar vesicles. Liposomes can be formed from various phospholipids, such as, for example, cholesterol, stearylamine or phosphatidylcholines.

The compounds and the salts, solvates, tautomers and stereoisomers thereof can also be delivered using monoclonal antibodies as individual carriers to which the compound molecules are coupled. The compounds can also be coupled to soluble polymers as targeted medicament carriers. Such polymers may encompass polyvinylpyrrolidone, pyran copolymer, polyhydroxypropyl-methacrylamidophenol, polyhydroxyethylaspartamidophenol or polyethylene oxide polylysine, substituted by palmitoyl radicals. The compounds may furthermore be coupled to a class of biodegradable polymers which are suitable for achieving controlled release of a medicament, for example polylactic acid, poly-epsilon-caprolactone, polyhydroxybutyric acid, poly-orthoesters, polyacetals, polydihydroxypyrans, polycyanoacrylates and crosslinked or amphipathic block copolymers of hydrogels.

Pharmaceutical formulations adapted for transdermal administration can be administered as independent plasters for extended, close contact with the epidermis of the recipient. Thus, for example, the active ingredient can be delivered from the plaster by iontophoresis, as described in general terms in Pharmaceutical Research, 3(6), 318 (1986).

Pharmaceutical compounds adapted for topical administration can be formulated as ointments, creams, suspensions, lotions, powders, solutions, pastes, gels, sprays, aerosols or oils.

For the treatment of the eye or other external tissue, for example mouth and skin, the formulations are preferably applied as topical ointment or cream. In the case of formulation to give an ointment, the active ingredient can be employed either with a paraffinic or a water-miscible cream base. Alternatively, the active ingredient can be formulated to give a cream with an oil-in-water cream base or a water-in-oil base.

Pharmaceutical formulations adapted for topical application to the eye include eye drops, in which the active ingredient is dissolved or suspended in a suitable carrier, in particular an aqueous solvent.

Pharmaceutical formulations adapted for topical application in the mouth encompass lozenges, pastilles and mouthwashes.

Pharmaceutical formulations adapted for rectal administration can be administered in the form of suppositories or enemas.

Pharmaceutical formulations adapted for nasal administration in which the carrier substance is a solid comprise a coarse powder having a particle size, for example, in the range 20-500 microns, which is administered in the manner in which snuff is taken, i.e. by rapid inhalation via the nasal passages from a container containing the powder held close to the nose. Suitable formulations for administration as nasal spray or nose drops with a liquid as carrier substance encompass active-ingredient solutions in water or oil.

Pharmaceutical formulations adapted for administration by inhalation encompass finely particulate dusts or mists, which can be generated by various types of pressurised dispensers with aerosols, nebulisers or insufflators.

Pharmaceutical formulations adapted for vaginal administration can be administered as pessaries, tampons, creams, gels, pastes, foams or spray formulations.

Pharmaceutical formulations adapted for parenteral administration include aqueous and non-aqueous sterile injection solutions comprising antioxidants, buffers, bacteriostatics and solutes, by means of which the formulation is rendered isotonic with the blood of the recipient to be treated; and aqueous and non-aqueous sterile suspensions, which may comprise suspension media and thickeners. The formulations can be administered in single-dose or multidose containers, for example sealed ampoules and vials, and stored in freeze-dried (lyophilised) state, so that only the addition of the sterile carrier liquid, for example water for injection purposes, immediately before use is necessary. Injection solutions and suspensions prepared in accordance with the recipe can be prepared from sterile powders, granules and tablets.

It goes without saying that, in addition to the above particularly mentioned constituents, the formulations may also comprise other agents usual in the art with respect to the particular type of formulation; thus, for example, formulations which are suitable for oral administration may comprise flavours.

A therapeutically effective amount of a compound depends on a number of factors, including, for example, the age and weight of the animal, the precise condition that requires treatment, and its severity, the nature of the formulation and the method of administration, and is ultimately determined by the treating doctor or vet. However, an effective amount of a compound according to the invention is generally in the range from 0.1 to 100 mg/kg of body weight of the recipient (mammal) per day and particularly typically in the range from 1 to 10 mg/kg of body weight per day. Thus, the actual amount per day for an adult mammal weighing 70 kg is usually between 70 and 700 mg, where this amount can be administered as a single dose per day or usually in a series of part-doses (such as, for example, two, three, four, five or six) per day, so that the total daily dose is the same. An effective amount of a salt, solvate, tautomer and stereoisomer thereof can be determined as the fraction of the effective amount of the compound according to the invention *per se.* It can be assumed that similar doses are suitable for the treatment of other conditions mentioned above.

A combined treatment of this type can be achieved with the aid of simultaneous, consecutive or separate dispensing of the individual components of the treatment. Combination products of this type employ the compounds according to the invention.
The anti-cancer treatment defined herein may be applied as a sole therapy or may involve, in addition to the composition of the invention, conventional surgery or radiotherapy.

"Treating" as used herein, means an alleviation, in whole or in part, of symptoms associated with a disorder or disease, or slowing, or halting of further progression or worsening of those symptoms, or prevention or prophylaxis of the disease or disorder in a subject at risk for developing the disease or disorder.

The term "effective amount" in connection with a compound can mean an amount capable of alleviating, in whole or in part, symptoms associated with a disorder or disease, or slowing or halting further progression or worsening of those symptoms, or preventing or providing prophylaxis for the disease or disorder in a subject having or at risk for developing a disease disclosed herein, such as cancer,
The term "therapeutically effective" or "therapeutically effective amount" refers to an amount of a drug effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the cancer. To the extent the drug may prevent growth and/or kill existing cancer cells, it may be cytostatic and/or cytotoxic. For cancer therapy, efficacy can, for example, be measured by assessing the time to disease progression (TTP) and/or determining the response rate (RR).

Preferably, erlotinib, cetuximab, aflibercept, bevacizumab are administered once a week, preferably intravenously as infusion. Preferably the initial dose is 100 to 1000 mg per m² body surface, particurlarly preferably between 200 and 600 mg per m² body surface. Subsequent doses are 50 to 600 mg per m² body surface, particurlarly preferably between 100 and 400 mg per m² body surface.

### USE

The present compounds are suitable as pharmaceutical active ingredients for mammals, especially for humans, in the treatment of immune modulatory and stress response kinase-induced diseases. These diseases include neoplastic malignancies including, solid tumor cancers, cancers of the lymphatic or blood system, the proliferation of tumour cells, pathological neovascularisation (or angiogenesis) which promotes the growth of solid tumours, neurodegenerative diseases (Alzheimer, demyelinating core disorders multiple sclerosis), immune related disorders like arthritis, psoriasis, lupus, or other autoimmune diseases as well as chronic infections.

The present invention encompasses the use of the compounds and/or physiologically acceptable salts and solvates thereof for the preparation of a medicament for the treatment or prevention of cancer. Preferred carcinomas for the treatment originate from the group cerebral carcinoma, urogenital tract carcinoma, carcinoma of the lymphatic system, stomach carcinoma, laryngeal carcinoma and lung carcinoma. A further group of preferred forms of cancer are monocytic leukaemia, lung adenocarcinoma, small-cell lung carcinomas, pancreatic cancer, glioblastomas, melanomas and breast carcinoma. A further group of preferred forms of cancer include, cervical cancer, neuroblastoma, testicular cancer, macroglobulinemia and sarcomas. The present invention specifically relates to compounds and pharmaceutically acceptable salts, solvates, tautomers and stereoisomers thereof, including mixtures thereof in all ratios, for the use for the treatment of neoplastic malignancies (solid tumor cancers, cancers of the lymphatic or blood system), of neurodegenerative diseases, immune related disorders like arthritis, psoriasis, lupus, multiple sclerosis or other autoimmune diseases as well as chronic infections.

Especial preference is given to the use for the treatment of a disease where the disease is a neoplastic malignancies.

The neoplastic malignancies is preferably selected from the group of tumours of the lung, squamous epithelium, the bladder, the stomach, the kidneys, of head and neck, the oesophagus, the cervix, the thyroid, the intestine, the liver, the brain, the prostate, the urogenital tract, the lymphatic system, the stomach and/or the larynx.

The neoplastic malignancies is furthermore preferably selected from the group lung adenocarcinoma, small-cell lung carcinomas, pancreatic cancer, glioblastomas, colon carcinoma and breast carcinoma.

Preference is furthermore given to the use for the treatment of a neoplastic malignancies of the blood and immune system, preferably for the treatment of a tumour selected from the group of acute myeloid leukaemia, chronic myeloid leukaemia, acute lymphatic leukaemia and/or chronic lymphatic leukaemia.

Representative cancers that compounds are useful for treating or preventing include, cancer of the head, neck, eye, mouth, throat, esophagus, bronchus, larynx, pharynx, chest, bone, lung, colon, rectum, stomach, prostate, urinary bladder, uterine, cervix, breast, ovaries, testicles or other reproductive organs, skin, thyroid, blood, lymph nodes, kidney, liver, pancreas, brain, central nervous system, solid tumors and blood-borne tumors.

Moreover, the present invention specifically relates to compounds for the use for the treatment and/or prevention of cancer,
where the cancer to be treated is a solid tumour or a tumour of the blood and immune system.

Moreover, the present invention specifically relates to compounds, for the use for the treatment and/or prevention of cancer, where the where the tumour originates from the group of acute myeloid leukaemia, chronic myeloid leukaemia, acute lymphatic leukaemia and/or chronic lymphatic leukaemia.

Moreover, the present invention specifically relates to compounds, for the use for the treatment and/or prevention of cancer, where the solid tumour originates from the group of tumours of the epithelium, the bladder, the stomach, the kidneys, of head and neck, the esophagus, the cervix, the thyroid, the intestine, the liver, the brain, the prostate, the uro-genital tract, the lymphatic system, the stomach, the larynx, the bones, including chondosarcoma and Ewing sarcoma, germ cells, including embryonal tissue tumours, and/or the lung, from the group of monocytic leukaemia, lung adenocarcinoma, small-cell lung carcinomas, pancreatic cancer, glioblastomas, neurofibroma, angiosarcoma, breast carcinoma and /or maligna melanoma.

The disclosed compounds of can be administered in combination with other known therapeutic agents, including anticancer agents. As used here, the term "anticancer agent" relates to any agent which is administered to a patient with cancer for the purposes of treating the cancer.

Combination with MEK inhibitor N-((S)-2,3-dihydroxy-propyl)-3-(2-fluoro-4-iodo-phenylamino)-isonicotinamide in a human KP-4 pancreatic tumor models:
Method: Female BaIB/c nude mice (6-8 week old) where subcutaneously injected with human KP-4 pancreatic tumor cells and were divided into treatment groups (ten animals in one group) after the tumors were established. Respective groups were administered orally with the 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate (100 mg/kg) or N-((S)-2,3-dihydroxy-propyl)-3-(2-fluoro-4-iodo-phenylamino)-isonicotinamide (50 mg/kg) for 5 days on and 2 days off in monotherapy or in combination. At the end of treatment T/C values were calculated and tumor regrowth was observed. Statistical analysis has been done with one way ANOVA. Results: 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate and N-((S)-2,3-dihydroxy-propyl)-3-(2-fluoro-4-iodo-phenylamino)-isonicotinamide showed anti-tumor activity with T/C values of 35% and 42%, respectively. Combining both agents led to significant enhanced anti-tumor activity with a T/C of 14% (P<0.05). All treatments were well tolerated.

Combination with MEK inhibitor N-((S)-2,3-dihydroxy-propyl)-3-(2-fluoro-4-iodo-phenylamino)-isonicotinamide in 10 patient derived pancreatic tumor models:
Method: Female NMRI nude mice (6-8 week old) where subcutaneously implanted with a patient derived pancreatic tumor fragment. Animals were devided into treatments groups (ten animals in one group) after the tumors were established. Respective groups were administered orally with the 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate (100 mg/kg) or N-((S)-2,3-dihydroxy-propyl)-3-(2-fluoro-4-iodo-phenylamino)-isonicotinamide (100 mg/kg, MTD) for 5 days on and 2 days off in monotherapy or in combination. At the end of treatment T/C values were calculated and tumor regrowth was observed. Statistical analysis has been done with one way ANOVO. Results: 3-(1-{3-[5-(1-methyl-piperidin-4-ylmethoxy)-pyrimidin-2-yl]-benzyl)-6-oxo-1,6-dihydro-pyridazin-3-yl)-benzonitrile hydrochloride hydrate showed anti-tumor activity in 2/10 model with T/C values of 43% and 50% whereas N-((S)-2,3-dihydroxy-propyl)-3-(2-fluoro-4-iodo-phenylamino)-isonicotinamide showed anti-tumor activity in all 10 models (T/C values < 55%). Combination of both agents showed enhanced anti-tumor activity in all models where 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate monotherapy versus combination therapy was statistically significant in 10/10 models (P<0.01) and N-((S)-2,3-dihydroxy-propyl)-3-(2-fluoro-4-iodo-phenylamino)-isonicotinamide monotherapy versus combination was statistically significant in 2/10 models (P<0.05). All treatments were well tolerated.

## Claims

1. A composition comprising 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof and N-((S)-2,3-dihydroxy-propyl)-3-(2-fluoro-4-iodo-phenylamino)-isonicotinamide or a pharmaceutically acceptable salt and/or solvate thereof.

2. A composition comprising 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate and N-((S)-2,3-dihydroxy-propyl)-3-(2-fluoro-4-iodo-phenylamino)-isonicotinamide or a pharmaceutically acceptable salt and/or solvate thereof.

3. A composition according to claim 1 or 2, further comprising pharmaceutically acceptable excipients and/or adjuvants.

4. A composition of 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof and N-((S)-2,3-dihydroxy-propyl)-3-(2-fluoro-4-iodo-phenylamino)-isonicotinamide or a pharmaceutically acceptable salt and/or solvate thereof, for the use for the treatment of diseases selected from the group
cancer of the head, neck, eye, mouth, throat, esophagus, bronchus, larynx, pharynx, chest, bone, lung, colon, rectum, stomach, prostate, urinary bladder, uterine, cervix, breast, ovaries, testicles or other reproductive organs, skin, thyroid, blood, lymph nodes, kidney, liver, pancreas, brain, central nervous system, solid tumors and blood-borne tumors.

5. 3-(1-{3-[5-(1-methyl-piperidin-4-ylmethoxy)-pyrimidin-2-yl]-benzyl 6-oxo-1,6-dihydro-pyridazin-3-yl)-benzonitrile or a pharmaceutically acceptable salt and/or solvate thereof for use as a medicament for the treatment of cancer, selected from the group colorectal, lung, breast, kidney, and glioblastomas,
wherein the medicament is to be used in combination with N-((S)-2,3-dihydroxy-propyl)-3-(2-fluoro-4-iodo-phenylamino)-isonicotinamide or a pharmaceutically acceptable salt and/or solvate thereof.

6. 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate for use as a medicament for the treatment of cancer, selected from the group
colorectal, lung, breast, kidney, and glioblastomas,
wherein the medicament is to be used in combination with N-((S)-2,3-dihydroxy-propyl)-3-(2-fluoro-4-iodo-phenylamino)-isonicotinamide or a pharmaceutically acceptable salt and/or solvate thereof.

7. 3-(1-{3-[5-(1-methyl-piperidin-4-ylmethoxy)-pyrimidin-2-yl]-benzyl}-6-oxo-1,6-dihydro-pyridazin-3-yl)-benzonitrile or a pharmaceutically acceptable salt and/or solvate thereof or 3-(1-{3-[5-(1-methyl-piperidin-4-ylmethoxy)-pyrimidin-2-yl]-benzyl}-6-oxo-1,6-dihydro-pyridazin-3-yl)-benzonitrile hydrochloride hydrate for use according to claims 5 or 6, wherein the compound is administered to a patient in an amount of 250 mg to 12500 mg per week.

## Patentansprüche

1. Zusammensetzung enthaltend 3-(1-{3-[5-(1-Methyl-piperidin-4-yl-methoxy)-pyrimidin-2-yl]-benzyl}-6-oxo-1,6-dihydro-pyridazin-3-yl)-benzonitril oder ein pharmazeutisch unbedenkliches Salz und/oder Solvat davon und N-((S)-2,3-Dihydroxy-propyl)-3-(2-fluor-4-iod-phenylamino)-isonicotinamid oder ein pharmazeutisch unbedenkliches Salz und/oder Solvat davon.

2. Zusammensetzung enthaltend 3-(1-{3-[5-(1-Methyl-piperidin-4-yl-methoxy)-pyrimidin-2-yl]-benzyl}-6-oxo-1,6-dihydro-pyridazin-3-yl)-benzonitril-hydrochlorid-hydrat und N-((S)-2,3-Dihydroxy-propyl)-3-(2-fluor-4-iod-phenylamino)-isonicotinamid oder ein pharmazeutisch unbedenkliches Salz und/oder Solvat davon.

3. Zusammensetzung nach Anspruch 1 oder 2, weiterhin enthaltend pharmazeutisch unbedenkliche Träger- und/oder Hilfsstoffe.

4. Zusammensetzung aus 3-(1-{3-[5-(1-Methyl-piperidin-4-ylmethoxy)-pyrimidin-2-yl]-benzyl}-6-oxo-1,6-dihydro-pyridazin-3-yl)-benzonitril oder einem pharmazeutisch unbedenklichen Salz und/oder Solvat davon und N-((S)-2,3-Dihydroxy-propyl)-3-(2-fluor-4-iod-phenylamino)-isonicotinamid oder einem pharmazeutisch unbedenklichen Salz und/oder Solvat davon, zur Verwendung für die Behandlung von Krankheiten, die aus der Gruppe Krebs des Kopfes, Halses, Auges, Mundes, der Kehle, Speiseröhre, Bronchie, des Kehlkopfes, der Rachenhöhle, des Brustkorbes, Knochens, der Lunge, des Kolons, Rektums, Magens, der Prostata, Harnblase, Gebärmutter, des Muttermundes, der Brust, Eierstöcke, Hoden oder anderer Fortpflanzungsorgane, der Haut, Schilddrüse, des Blutes, der Lymphknoten, Niere, Leber, Bauchspeicheldrüse, des Hirns, Zentralnervensystems, feste Tumore und hämatogene Tumore ausgewählt sind.

5. 3-(1-{3-[5-(1-Methyl-piperidin-4-ylmethoxy)-pyrimidin-2-yl]-benzyl}-6-oxo-1,6-dihydro-pyridazin-3-yl)-benzonitril oder ein pharmazeutisch unbedenkliches Salz und/oder Solvat davon zur Verwendung als Arzneimittel für die Behandlung von Krebs, ausgewählt aus der Gruppe Kolorektal-, Lungen-, Brust-, Nierenkrebs und Glioblastome,
wobei das Arzneimittel in Kombination mit N-((S)-2,3-Dihydroxy-propyl)-3-(2-fluor-4-iod-phenylamino)-isonicotinamid oder einem pharmazeutisch unbedenklichen Salz und/oder Solvat davon zu verwenden ist.

6. 3-(1-{3-[5-(1-Methyl-piperidin-4-ylmethoxy)-pyrimidin-2-yl]-benzyl}-6-oxo-1,6-dihydro-pyridazin-3-yl)-benzonitril-hydrochlorid-hydrat zur Verwendung als Arzneimittel für die Behandlung von Krebs, ausgewählt aus der Gruppe
Kolorektal-, Lungen-, Brust-, Nierenkrebs und Glioblastome,
wobei das Arzneimittel in Kombination mit N-((S)-2,3-Dihydroxy-propyl)-3-(2-fluor-4-iod-phenylamino)-isonicotinamid oder einem pharmazeutisch unbedenklichen Salz und/oder Solvat davon zu verwenden ist.

7. 3-(1-{3-[5-(1-Methyl-piperidin-4-ylmethoxy)-pyrimidin-2-yl]-benzyl}-6-oxo-1,6-dihydro-pyridazin-3-yl)-benzonitril oder ein pharmazeutisch unbedenkliches Salz und/oder Solvat davon
oder 3-(1-{3-[5-(1-Methyl-piperidin-4-ylmethoxy)-pyrimidin-2-yl]-benzyl}-6-oxo-1,6-dihydro-pyridazin-3-yl)-benzonitril-hydrochlorid-hydrat zur Verwendung nach Ansprüchen 5 oder 6, wobei die Verbindung einem Patienten in einer Menge von 250 mg bis 12500 mg pro Woche verabreicht wird.

## Revendications

1. Composition comprenant du 3-(1-{3-[5-(1-méthylpipéridin-4-ylméthoxy)-pyrimidin-2-yl]-benzyl}-6-oxo-1,6-dihydropyridazin-3-yl)benzonitrile ou un sel et/ou solvate pharmaceutiquement acceptable de celui-ci et du N-((S)-2,3-dihydroxypropyl)-3-(2-fluoro-4-iodophénylamino)isonicotinamide ou un sel et/ou solvate pharmaceutiquement acceptable de celui-ci.

2. Composition comprenant du chlorhydrate de 3-(1-{3-[5-(1-méthylpipéridin-4-ylméthoxy)-pyrimidin-2-yl]-benzyl}-6-oxo-1,6-dihydropyridazin-3-yl)-benzonitrile hydraté et du N-((S)-2,3-dihydroxypropyl)-3-(2-fluoro-4-iodophénylamino)isonicotinamide ou un sel et/ou solvate pharmaceutiquement acceptable de celui-ci.

3. Composition selon la revendication 1 ou 2, comprenant en outre des excipients et/ou des adjuvants pharmaceutiquement acceptables.

4. Composition de 3-(1-{3-[5-(1-méthylpipéridin-4-ylméthoxy)pyrimidin-2-yl]-benzyl}-6-oxo-1,6-dihydropyridazin-3-yl)benzonitrile ou d'un sel et/ou solvate pharmaceutiquement acceptable de celui-ci et de N-((S)-2,3-dihydroxypropyl)-3-(2-fluoro-4-iodophénylamino)isonicotinamide ou d'un sel et/ou solvate pharmaceutiquement acceptable de celui-ci, pour une utilisation dans le traitement de maladies choisies dans le groupe constitué par le cancer de la tête, du cou, de l'oeil, de la bouche, de la gorge, de l'oesophage, des bronches, du larynx, du pharynx, de la poitrine, des os, du poumon, du côlon, du rectum, de l'estomac, de la prostate, de la vessie urinaire, de l'utérus, du col de l'utérus, du sein, des ovaires, des testicules ou autres organes reproducteurs, de la peau, de la thyroïde, du sang, des noeuds lymphoïdes, du rein, du foie, du pancréas, du cerveau, du système nerveux central, les tumeurs solides et les tumeurs du sang.

5. 3-(11-{3-[5-(1-Méthylpipéridin-4-ylméthoxy)pyrimidin-2-yl]-benzyl}-6-oxo-1,6-dihydropyridazin-3-yl)benzonitrile ou un sel et/ou solvate pharmaceutiquement acceptable de celui-ci pour une utilisation comme médicament dans le traitement du cancer, choisi dans le groupe constitué par le cancer colorectal, du poumon, du sein, du rein, et les glioblastomes,
où le médicament doit être utilisé en combinaison avec du N-((S)-2,3-dihydroxypropyl)-3-(2-fluoro-4-iodophénylamino)isonicotinamide ou un sel et/ou solvate pharmaceutiquement acceptable de celui-ci.

6. Chlorhydrate de 3-(1-{3-[5-(1-méthylpipéridin-4-ylméthoxy)pyrimidin-2-yl]-benzyl}-6-oxo-1,6-dihydropyridazin-3-yl)-benzonitrile hydraté pour une utilisation comme médicament dans le traitement du cancer, choisi dans le groupe constitué par le cancer colorectal, du poumon, du sein, du rein, et les glioblastomes,
où le médicament doit être utilisé en combinaison avec du N-((S)-2,3-dihydroxypropyl)-3-(2-fluoro-4-iodophénylamino)isonicotinamide ou un sel et/ou solvate pharmaceutiquement acceptable de celui-ci.

7. 3-(1-{3-[5-(1-Méthylpipéridin-4-ylméthoxy)pyrimidin-2-yl]-benzyl}-6-oxo-1,6-dihydropyridazin-3-yl)-benzonitrile ou un sel et/ou solvate pharmaceutiquement acceptable de celui-ci
ou
chlorhydrate de 3-(1-{3-[5-(1-méthylpipéridin-4-ylméthoxy)pyrimidin-2-yl]-benzyl}-6-oxo-1,6-dihydropyridazin-3-yl)-benzonitrile hydraté pour une utilisation selon les revendications 5 ou 6, où le composé est administré à un patient selon une quantité allant de 250 mg à 12 500 mg par semaine.
